# EUROPEAN PATENT SPECIFICATION

(11) **EP 4 168 382 B1**
(45) Date of publication and mention of the grant of the patent: **01.05.2024**
(21) Application number: 21736013.0
(22) Date of filing: 15.06.2021
(51) Int. Cl.: C07C 51/09

(54) **FIBRES**
FASERN
FIBRES

(30) Priority: 17.06.2020 GB 202009216
(43) Date of publication of application: 26.04.2023
(73) Proprietor: CPL Products Limited, Sheffield S21 1TZ (GB); The University of Nottingham, Nottingham NG7 2RD (GB)
(72) Inventor: GILL, Andrew, Sheffield S21 1TZ (GB); SNAPE, Colin, Nottingham University Park Nottingham NG7 2RD (GB)
(74) Representative: HGF
(86) International application number: PCT/GB2021/051486
(87) International publication number: WO 2021/255427

(56) References cited:
- WO-A1-2020/013755
- CN-A- 106 519 293
- US-A1- 2019 218 362
- Anna Palme ET AL: "Development of an efficient route for combined recycling of PET and cotton from mixed fabrics", Textiles and Clothing Sustainability, 1 January 2017 (2017-01-01), pages 1-9, XP055675406, DOI: 10.1186/s40689-017-0026-9 Retrieved from the Internet: URL:https://textclothsustain.springeropen. com/articles/10.1186/s40689-017-0026-9 [retrieved on 2020-03-10]
- LING CHEN ET AL: "Separation of waste polyester/cotton blended fabrics by phosphotungstic acid and preparation of terephthalic acid", POLYMER DEGRADATION AND STABILITY, vol. 161, 22 January 2019 (2019-01-22), pages 157-165, XP085625350, ISSN: 0141-3910, DOI: 10.1016/J.POLYMDEGRADSTAB.2019.01.022

## Description

This invention relates generally to processing fibres. More specifically, although not exclusively, this invention relates to a process for chemically recycling fabric or textiles waste.

The amount of clothing bought per person in the EU has increased by 40% in the past few decades, driven by a fall in price and the increased speed with which fashion is delivered to consumers. Once discarded, over half the garments are not recycled, but end up in mixed household waste and are subsequently sent to incinerators or landfill (European Parliament Briefing PE 633.143 "Environmental impact of the textile and clothing industry", January 2019). Therefore, there is a significant impact on the environment caused by clothing waste.

Many garments are fabricated from cotton, polyester, or a mixture of both.

It is known to recycle cotton clothing to produce recycled cotton fibres. However, there are some notable limitations such as fibre length being shorter, which leads to a reduction in the durability of resulting garments fabricated from recycled cotton. In addition, the production of cotton is resource-intensive despite it being a natural and biodegradable fibre. Therefore, the production and waste of cotton has a considerable environmental impact.

Polyester is a synthetic fibre made from fossil fuels. However, it does have a number of advantages, including its ability to be washed effectively at lower temperatures in comparison to natural fibres. Polyester also dries more quickly and rarely needs ironing, which reduces the amount of energy required to launder garments.

According to the European Parliament Briefing "Environmental impact of the textile and clothing industry" (supra), only 1% of clothing is recycled into new clothes, since technologies that would enable recycling clothes into usable fibres are only starting to emerge.

Polyester (i.e. polyethylene terephthalate (PET)) is a condensation polymer formed from the reaction of ethylene glycol with terephthalic acid or dimethyl terephthalate. Several methods have been proposed for its recycling.

One approach for recycling polyester is to hydrolyse scrap polyethylene terephthalate into useful constituents. US4973746 describes a process for converting PET scrap into diamine monomers such as para-xylylenediamine, 1,4-bis(aminomethyl)cyclohexane, and 4-aminomethyl benzoic acid, which may be used in the production of various polyamides. The first step of the process involves ammonolysis of PET to produce terephthalamide and ethylene glycol. Spychaj et al. (Ind. Eng. Chem. Res. 1997, 36 1373) describes the neutral hydrolysis of PET into ethylene glycol and terephthalic acid in water at 200 to 280 °C. The crude product was further purified by heating with water at 310 to 370 °C.

It will be appreciated that the above simply details recycling scrap PET. In most recycling centres, significant effort is deployed in separating textiles into natural and man-made fibres.

US2019218362 discloses systems and methods that involve a subcritical water reaction to recycle the cellulose and polyester components of waste cotton and cotton/polyester blend textiles that would otherwise be discarded or disposed of. Palme et al. (Textiles and Clothing Sustainability, 2017, 1-9) discloses development of an efficient route for combined recycling of PET and cotton from mixed fabrics. Ling et al. (Polymer Degradation and Stability, 161, 2019, 157-165) discloses separation of waste polyester/cotton blended fabrics by phosphotungstic acid and preparation of terephthalic acid. WO2020/013755 discloses a process for separation of the cellulosic part from a raw material composition comprising polyester and cellulose containing composition, wherein the process comprises providing a blend, wherein the blend comprises a raw material composition and a hydrolyzing liquor. CN106519293 discloses a waste polyester-cotton textile degrading and recycling process and system.

It is therefore a first non-exclusive object of the invention to provide a process for extracting useful chemical constituents from waste fabric and textiles containing mixed materials.

Accordingly, a first aspect of the invention provides a process for processing a feedstock containing cotton fibres and PET fibres, the process comprising:
i. heating a suspension of feedstock containing cotton fibres and PET fibres in water at a temperature above 180 °C to produce a mixture comprising char;
ii. removing the water from the mixture comprising char;
iii. adding a base to the mixture comprising char to produce a terephthalate salt in a liquid phase;
iv. removing the char from the liquid phase to produce dried char;
v. acidifying the liquid phase to produce terephthalic acid.

Advantageously, the method of the invention allows for the processing of mixed, e.g. mixed coloured, feedstock without prior sorting into natural and man-made fibres. One or both of the cotton fibres and/or PET fibres may comprise or consist of coloured fibres, e.g. dyed fibres.

Preferably, the feedstock is fabric or textiles, for example, waste fabric or waste textiles. The feedstock containing cotton fibres and PET fibres may comprise or consist of both cotton and PET fibres, e.g. mixed polyester and cotton clothing waste. In an embodiment, the feedstock comprises fabric waste in a 70:30 cotton to PET ratio (w/w). However, the ratio of cotton to PET (or PET to cotton) in the feedstock may be, for example, 20:80, 40:60, 50:50, 60:40, 70:30, 80:20, or 90:10 (w/w).

In the process of the invention, the cotton of the feedstock may be converted into char in a hydrothermal carbonisation process. The char may be further processed to produce a solid fuel, e.g. bio-coal. These steps are advantageously performed in the presence of PET in the feedstock. The process of the invention enables the PET to be hydrolysed into terephthalic acid and ethylene glycol. Advantageously, the terephthalic acid is recovered in very high purity, say, of greater than 99.9%, which may be sold for re-use in further PET manufacture. Therefore, the invention provides a process to convert mixed polyester and cotton clothing waste to produce solid fuel and to recover PET monomers.

Moreover, solid fuel, e.g. bio-coal, is considerably cheaper than wood pellets for the small to medium scale biomass boiler market. Advantageously, the biocoal produced in the process of the invention contains similar levels of ash to wood pellets unlike many other biomass derived fuels which are usually higher.

In embodiments, the fabric waste may be shredded prior to Step i.. The fabric waste may be shredded into pieces with a length or diameter of less than 50mm, e.g. less than 40mm, less than 30mm, or less than 20mm. In embodiments, the shredded pieces may have a length or diameter of less than 15mm, or less than 10mm. It is preferable that the fabric waste has the smallest pieces possible.

In embodiments, Step i. of the process may comprise heating a suspension of feedstock in water in a volume to weight (v/w) ratio of between 4:1 to 8:1, e.g. 4:1, 5:1, 6:1, 7:1, or 8:1, water to feedstock.

In embodiments, in Step i., the feedstock may be heated to a temperature between 180 to 240 °C, for example, between 190 to 230 °C, or between 200 to 220 °C. The pressure of Step i. may be greater than 1.5 x 10⁶ Pa (15 bar). For example, the pressure to which the feedstock is exposed in Step i. may be greater than 2.0 x 10⁶ Pa (20 bar), or greater than 2.5 x 10⁶ Pa (25 bar). The pressure may be between 2.0 x 10⁶ Pa (20 bar) and 2.5 x 10⁶ Pa (25 bar), e.g. 2.3 x 10⁶ Pa (23 bar).

In embodiments, Step ii. comprises removing the water from the mixture comprising char via filtration. Additionally or alternatively, Step ii. may comprise removing the water from the mixture comprising char via centrifugation.

Advantageously, the water removed from the mixture comprising char may be reused in the process, e.g. in Step i. of the process.

In embodiments, the process may further comprise recovering ethylene glycol from the water removed in Step ii. of the process. This may be performed via distillation.

In embodiments, Step iii. may comprise adding a base to the mixture comprising char. In embodiments, Step iii. may comprise adding a base comprising ammonia and/or ammonium hydroxide to the mixture comprising char to produce diammonium terephthalate in a liquid phase. In embodiments, Step iii. may additionally or alternatively comprise adding a base, for example a base comprising a metal hydroxide to the mixture comprising char to produce a di-metal terephthalate salt in a liquid phase. For example, in embodiments, Step iii. may additionally or alternatively comprise adding a base comprising sodium hydroxide to the mixture comprising char to produce di-sodium terephthalate in a liquid phase.

In embodiments, Step iii. is performed at a temperature between 0 to 40 °C, or between 5 to 35 °C, or between 10 to 30 °C, or between 15 to 25 °C. In an embodiment, Step iii. is performed at room temperature. This is advantageous because no heat energy is required for Step iii., which increases the energy efficiency of the process.

In embodiments, Step iv. removing the char from the liquid phase may comprise filtering the char from the liquid phase, for example, using a filter press.

The process may further comprise drying the char to produce a solid fuel. In embodiments, the process may further comprise forming pellets and/or briquettes from the char to form a solid fuel. For example, the char may be compressed with or without a binder and/or other waste materials to form pellets and/or briquettes.

In embodiments, the process may further comprise Step vi. purifying the liquid phase by contacting the liquid phase with one or more of activated carbon and/or clay, e.g. bentonites and/or acid activated clay. In embodiments, Step vi. is performed after Step iii. and before Step v.

Advantageously, the activated carbon, e.g. activated charcoal, aids the removal of impurities, for example, dyes, humic acid, organic impurities, and/or other impurities from the liquid phase containing a terephthalate salt and/or terephthalic acid. In addition or alternatively, clay may be used to remove humic acid. The use of a clay beneficially enables the terephthalic acid to be recovered at a higher purity in the process of the invention.

In embodiments, the activated carbon and/or clay may be removed via filtration. Advantageously, in embodiments the activated carbon and/or the clay may be regenerated for re-use in the process.

In embodiments, Step v. of the process may comprise acidifying the liquid phase to produce terephthalic acid using an acid, for example hydrochloric acid, e.g. concentration HCl. In embodiments, the pH may be reduced to less than or equal to 5.5, for example, less than or equal to one of 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, or 2.0. For example, the pH may be reduced to between 5.5 to 1.5, for example, between 5.0 to 2.0, or between 4.0 to 3.0. In this step, the crude terephthalate salt precipitates out to form pure terephthalic acid in greater than or equal to 99.9% purity. In embodiments, the pure terephthalic acid may be collected via filtration.

In embodiments, the process may further comprise Step vii. torrefaction of the mixture comprising char. In embodiments, Step vii. may be performed after Step ii. and before Step iii. of the process. In embodiments, Step vii. may be performed at a temperature between 200 to 280 °C, e.g. between 220 to 260°C, or between 240 to 250 °C. In embodiments, Step vii. is performed for less than one hour, e.g. 30 minutes.

It has been surprisingly found that performing Step vii. on the mixture comprising char provides a 30% uplift in energy value of the resulting solid fuel, in contrast to the same process in which Step vii. is not performed.

Advantageously, energy from the steam produced in the torrefaction step may be recovered.

More advantageously, the torrefaction step is particularly effective at removing humic acid from the char mixture. Humic acid is a deep purple colour, which is difficult to remove from feedstock containing coloured fibres. It is undesirable for terephthalic acid recovered from the process to contain impurities, which will harm the transparency and/or the desired colour of further PET products manufactured from the recovered terephthalic acid.

Most advantageously, the process according to the invention may be used for processing feedstock containing humic acid and coloured PET fibres containing dyes. Without wishing to be bound by any particular theory, the torrefaction step of the process removes humic acid, whilst the use of activated carbon and/or clay removes dyes from coloured PET fibres. Therefore, the process of the invention may be used to recover terephthalic acid in high purity from coloured feedstock. This is advantageous because the recovered high purity terephthalic acid may then be used in further manufacturing of transparent PET without containing any remnants of dyes or other coloured compounds that might otherwise impair the PET product.

In embodiments, the process may comprise Step vi. and Step vii. In embodiments, Step vi. may comprise using only activated carbon and not clay.

This is advantageous because there is no need to dispose of, or recycle, the clay used in Step vi. of this embodiment. It is also easier to recycle activate carbon alone rather than a mixture comprising activated carbon and clay. Moreover, the inventors have found that the liquid phase is easier to filter if only activated carbon is used with no clay.

Advantageously, the process of the invention is environmentally sound due to the use of only water as a solvent. In addition, the waste effluent may be recycled for use in the process.

There is also disclosed a method of processing a PET-containing feedstock, the method comprising:
a. heating a suspension of PET-containing feedstock in water at a temperature above 180 °C to produce a mixture;
b. adding a base to the mixture to produce a terephthalate salt in a liquid phase;
c. acidifying the liquid phase to produce terephthalic acid.

Advantageously, the method allows for the processing of solid PET, e.g. from plastic bottles or other food containers. The PET feedstock may be coloured e.g. mixed coloured, feedstock without prior sorting into specific colours or dye types.

The method of the invention enables the PET to be hydrolysed into terephthalic acid and ethylene glycol. Advantageously, the terephthalic acid is recovered in very high purity, say, of greater than 99.9%, which may be sold for re-use in further PET manufacture.

The PET feedstock may be shredded or otherwise cut into pieces, e.g. pieces having a width or diameter of between 1 to 10 cm, e.g. from 2 to 9 cm, or from 3 to 8 cm, or from 4 to 6 cm.

In embodiments, Step a. of the method may comprise heating a suspension of feedstock in water in a volume to weight (v/w) ratio of between 4:1 to 8:1, e.g. 4:1, 5:1, 6:1, 7:1, or 8:1, water to feedstock.

In embodiments, in Step a., the feedstock may be heated to a temperature between 180 to 240 °C, for example, between 190 to 230 °C, or between 200 to 220 °C. The pressure of Step a. may be greater than 1.5 x 10⁶ Pa (15 bar). For example, the pressure to which the feedstock is exposed in Step a. may be greater than 2.0 x 10⁶ Pa (20 bar), or greater than 2.5 x 10⁶ Pa (25 bar). The pressure may be between 2.0 x 10⁶ Pa (20 bar) and 2.5 x 10⁶ Pa (25 bar), e.g. 2.3 x 10⁶ Pa (23 bar).

In embodiments, the method may further comprise recovering ethylene glycol from the water of Step a. of the method. This may be performed via distillation.

In embodiments, Step b. may comprise adding a base to the mixture. In embodiments, Step b. may comprise adding a base comprising ammonia and/or ammonium hydroxide to the mixture to produce diammonium terephthalate in a liquid phase. In embodiments, Step b. may additionally or alternatively comprise adding a base comprising a metal hydroxide to the mixture to produce a di-metal terephthalate salt in a liquid phase. For example, in embodiments, Step b. may additionally or alternatively comprise adding a base comprising sodium hydroxide to the mixture to produce di-sodium terephthalate in a liquid phase.

In embodiments, Step b. is performed at a temperature between 0 to 40 °C, or between 5 to 35 °C, or between 10 to 30 °C, or between 15 to 25 °C. In an embodiment, Step b. is performed at room temperature. This is advantageous because no heat energy is required for Step b., which increases the energy efficiency of the method.

In embodiments, the method may further comprise Step d. purifying the liquid phase by contacting the liquid phase with one or more of activated carbon and/or clay, e.g. bentonites and/or acid activated clay. In embodiments, Step d. is performed after Step b. and before Step c.

Advantageously, the activated carbon, e.g. activated charcoal, aids the removal of impurities, for example, dyes, humic acid, organic impurities, and/or other impurities from the liquid phase containing a terephthalate salt and/or terephthalic acid. In addition or alternatively, clay may be used to remove humic acid. This enables the terephthalic acid to be recovered at a higher purity in the process of the invention.

In embodiments, the activated carbon and/or clay may be removed via filtration. Advantageously, in embodiments the activated carbon and/or the clay may be regenerated for re-use in the method.

In embodiments, Step c. of the method may comprise acidifying the liquid phase to produce terephthalic acid using an acid, for example hydrochloric acid, e.g. concentration HCl. In embodiments, the pH may be reduced to less than or equal to 5.5, for example, less than or equal to one of 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, or 2.0. For example, the pH may be reduced to between 5.5 to 1.5, for example, between 5.0 to 2.0, or between 4.0 to 3.0. In this step, the crude terephthalate salt precipitates out to form pure terephthalic acid in greater than or equal to 99.9% purity. In embodiments, the pure terephthalic acid may be collected via filtration.

The method of the invention may be used to recover terephthalic acid in high purity from coloured feedstock. This is advantageous because the recovered high purity terephthalic acid may then be used in further manufacturing of transparent PET without containing any remnants of dyes or other coloured compounds that might otherwise impair the PET product.

In embodiments, Step d. may comprise using only activated carbon and not clay.

This is advantageous because there is no need to dispose of, or recycle, the clay used in Step d. of this embodiment. It is also easier to recycle activate carbon alone rather than a mixture comprising activated carbon and clay. Moreover, the inventors have found that the liquid phase is easier to filter if only activated carbon is used with no clay.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. For the avoidance of doubt, the terms "may", "and/or", "e.g.", "for example" and any similar term as used herein should be interpreted as non-limiting such that any feature so-described need not be present. Indeed, any combination of optional features is expressly envisaged without departing from the scope of the invention, whether or not these are expressly claimed. The applicant reserves the right to change any originally filed claim or file any new claim accordingly, including the right to amend any originally filed claim to depend from and/or incorporate any feature of any other claim although not originally claimed in that manner.

Embodiments of the invention will now be described by way of example only with reference to the accompanying drawings in which:
Figure 1 is process for producing solid fuel and terephthalic acid from fabric waste containing cotton fibres and PET fibres, according to a first embodiment of the invention; and
Figure 2 is process for producing solid fuel and terephthalic acid from fabric waste containing cotton fibres and PET fibres, according to a second embodiment of the invention.

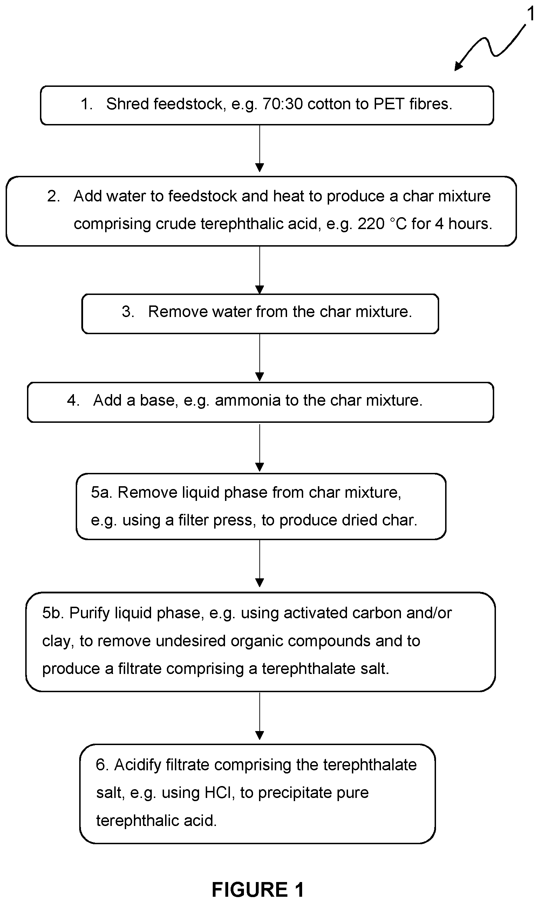

Referring now to Figure 1, there is shown a process 1 for producing solid fuel, e.g. bio-coal, and terephthalic acid from fabric waste containing cotton fibres and PET fibres, according to a first embodiment of the invention. The process 1 comprises six steps.

In Step 1 of the process, the feedstock is shredded into the smallest pieces possible, e.g. using a shredder. The feedstock is a mixture of cotton and PET (polyethylene terephthalate) fibres.

In Step 2, the shredded feedstock is converted into char. The shredded feedstock is added to a reactor and mixed with a suitable amount of water in a ratio of between 4:1 to 8:1 volume to weight of water to shredded feedstock. The shredded feedstock and water mixture is then subjected to an elevated temperature. The reaction conditions are 200 to 220 °C for 4 hours at 23 bar. In use, the reactor is a sealed vessel. A suitable reactor for use in this step is that described in EP2719748 B1 or EP2366757 B1.

After this step, the char may additionally contain monomers of the PET polymer, e.g. terephthalic acid and/or ethylene glycol.

In Step 3, the char is dewatered, i.e. the water is removed from the char. This may be performed using via filtration using a screen with a rinse. However, this step may also be performed using another suitable solid-liquid separation process such as centrifugation. The dewatering step may be a multistep process, e.g. filtering and subsequent centrifugation.

Advantageously, the process water removed from the char may optionally be reused in Step 2 of the process to add to more feedstock and heat in the reactor.

In embodiments of the process, ethylene glycol (originating from the PET fibres) may be recovered from the water phase. Advantageously, terephthalic acid is poorly soluble in water, and therefore remains associated with the char solid phase.

In Step 4, a base, e.g. ammonia, is added to the dewatered char. This may be performed at room temperature. In embodiments wherein the base is ammonia, the ammonia may be added to the char as ammonium hydroxide. This step results in solid char and a liquid phase containing di-ammonium terephthalate.

In Step 5a, the solid char is separated from the liquid phase, for example, using a filter press. The solid char is dried. In embodiments, the solid char may be pelletised or used to form briquettes for use as a fuel.

In Step 5b, the liquid phase is then purified. In addition to crude terephthalic acid in the form of di-ammonium terephthalate, the liquid phase may further contain dyes and other water-soluble organic molecules. In this embodiment, purification comprises contacting the liquid phase with activated carbon and/or clay (e.g. bentonites and/or acid activated clay) to remove said dyes and other water-soluble organic molecules. The activated carbon and/or clay is removed, e.g. via filtration, to provide a clear filtrate containing di-ammonium terephthalate.

Advantageously, the activated carbon and/or the clay may be regenerated for re-use in the process.

In Step 6, the pH of the clear filtrate containing di-ammonium terephthalic acid is reduced using concentrated hydrochloric acid to pH 5. In this step, the crude di-ammonium terephthalate precipitates out to form pure terephthalic acid in greater than or equal to 99.9% purity. This may be collected via filtration.

The waste effluent is preferably recycled.

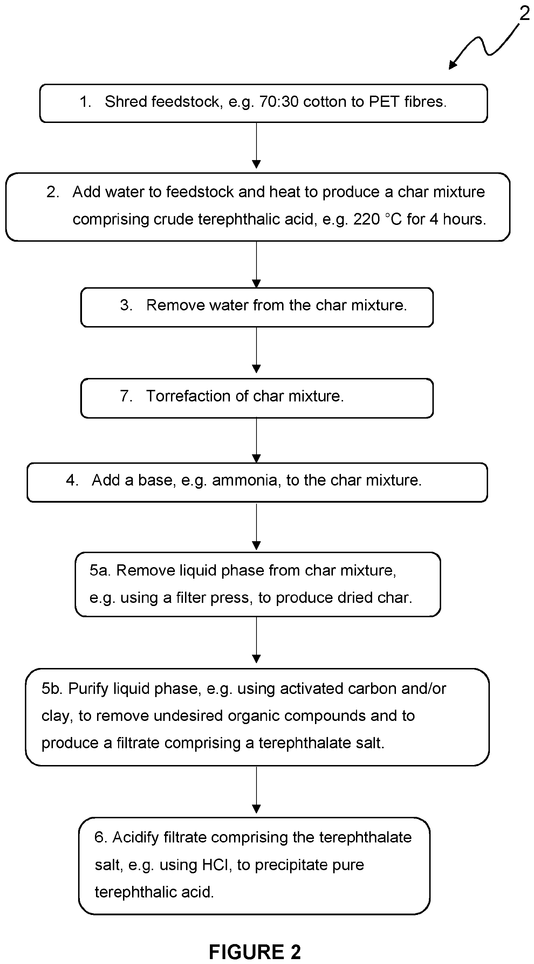

Referring now to Figure 2, there is shown a process 2 according to a second embodiment of the invention. The process 2 comprises many similar steps to the process 1 shown in Figure 1. Only the differences will be further described.

Steps 1 to 3 of the process are performed in a like-manner to that described previously for the process 1.

The process 2 comprises an additional step, Step 7, which is performed after Step 3. In Step 7, the char from Step 3 undergoes a torrefaction process. This is performed at 240 °C for 30 minutes.

Advantageously, energy from the steam produced in the torrefaction process may be recovered. More advantageously, the torrefaction process removes humic acid from the cotton component of the feedstock.

The torrefied char produced in Step 7 is then used in Steps 4 to 6, which are performed in a like-manner to that described previously for the process 1.

In this embodiment, Step 5 involves the use of activated carbon only, rather than using clay.

The invention will now be further described with reference to the following non-limiting Examples.

### Example 1 using Process 1 of the Invention

The feedstock comprised a 70:30 ratio of cotton to PET fabric. The feedstock was shredded into pieces having an average length of 15 mm. The shredded feedstock (6g) was added to a reactor with a capacity of 55 mL. Water (36 mL) was added to the reactor in a 6:1 mass ratio of water to feedstock. The mixture was heated for 4 hours at 220 °C to produce a char mixture in water. The mixture was cooled to room temperature and the water was removed using a screen. Ammonium hydroxide (25%) was added to the filtered char mixture. The solid char was removed from the liquid phase via filtration. The solid char was pelletised to produce fuel having an energy value of 19 MJ/kg. The yield of pelletised char was 50% on a dry basis.

Activated carbon and clay were contacted with the liquid phase. Once the liquid phase had turned colourless, the activated carbon and clay were removed via filtration. The resulting clear filtrate was acidified to pH 5 using HCl (12M). The precipitate was removed via filtration to produce terephthalic acid with a purity of 99.9% in a yield of 60%.

### Example 2 using Process 2 of the Invention

The feedstock comprised a 70:30 ratio of cotton to PET fabric. The feedstock was shredded into pieces having an average length of 15 mm. The shredded feedstock (6g) was added to a reactor with a capacity of 55 mL. Water (36 mL) was added to the reactor in a 6:1 mass ratio of water to feedstock. The mixture was heated for 4 hours at 220 °C to produce a char mixture in water. The mixture was cooled to room temperature and the water was removed using screen. The char mixture underwent a torrefaction process at 240 °C for 30 minutes.

Ammonium hydroxide (25%) was added to the filtered char mixture. The solid char was removed from the liquid phase via filtration. The solid char was pelletised to produce fuel having an energy value of 25 MJ/kg. The yield of pelletised char was 40% on a dry basis.

Activated carbon was added to the liquid phase. Once the liquid phase had turned colourless, the activated carbon and clay were removed via filtration. The resulting clear filtrate was acidified to pH 5 using HCl (12M). The precipitate was removed via filtration to produce terephthalic acid with a purity of 99.9% in an 89% yield.

It is shown that, although the yield of pelletised char in Example 2 is 20% lower than the yield of pelletised char in Example 1, the calorific value of the char in Example 2 is 30% higher than that produced in Example 1. Therefore, the solid char of Example 2 is a more valuable product.

It is thought that the torrefaction step removes the water from the char mixture to reduce the amount of water from 40 w/w% in the char mixture to 10 w/w% in the char prior to pelletising.

Additionally, torrefaction of the char mixture in Example 2 appears to at least partially remove the colour of the char mixture. This further aids removal of, for example, humic acid, dyes and other organic compounds in the process.

It will be appreciated by those skilled in the art that several variations to the aforementioned embodiments are envisaged without departing from the scope of the invention.

It will also be appreciated by those skilled in the art that any number of combinations of the aforementioned features and/or those shown in the appended drawings provide clear advantages over the prior art and are therefore within the scope of the invention described herein.

## Claims

1. A process (1, 2) for processing feedstock containing cotton fibres and PET fibres, the process (1,2) comprising:
i. heating a suspension of feedstock containing cotton fibres and PET fibres in water at a temperature above 180 °C to produce a mixture comprising char;
ii. removing the water from the mixture comprising char;
iii. adding a base to the mixture comprising char to produce a terephthalate salt in a liquid phase;
iv. removing the char from the liquid phase to produce dried char;
v. acidifying the liquid phase to produce terephthalic acid.

2. A process (1, 2) according to Claim 1, further comprising shredding the feedstock prior to Step i., e.g. into pieces having a length and/or diameter of less than 50 mm.

3. A process (1,2) according to any preceding Claim, wherein the ratio of water to feedstock is between 4:1 to 8:1 (w/w), e.g. 4:1, 5:1, 6:1, 7:1, or 8:1 (w/w), water to feedstock.

4. A process (1, 2) according to any preceding Claim, wherein Step i. comprises heating a suspension of feedstock containing cotton fibres and PET fibres in water at a temperature of between 180 to 240 °C.

5. A process (1, 2) according to any preceding Claim, wherein Step i. further comprises heating a suspension of feedstock containing cotton fibres and PET fibres in water under a pressure of greater than 2.0 x 10⁶ Pa (20 bar), e.g. 2.3 x 10⁶ Pa (23 bar).

6. A process (1, 2) according to any preceding Claim, wherein Step ii. comprises removing the water from the mixture comprising char via filtration.

7. A process (1, 2) according to any preceding Claim, further comprising recovering ethylene glycol from the water removed in Step ii. of the process (1, 2), e.g. via distillation.

8. A process (1, 2) according to any preceding Claim, wherein Step iii. comprises adding a base comprising ammonia and/or ammonium hydroxide to the mixture comprising char to produce diammonium terephthalate in a liquid phase, wherein Step iii. may be performed at room temperature, e.g. between 0 to 40 °C, or between 5 to 35 °C, or between 10 to 30 °C, or between 15 to 25 °C.

9. A process (1, 2) according to any preceding Claim, wherein Step iv. comprises filtering the char from the liquid phase, for example, using a filter press.

10. A process (1, 2) according to any preceding Claim, further comprising drying the char to produce a solid fuel.

11. A process (1, 2) according to any preceding Claim, further comprising forming pellets and/or briquettes from the char to form a solid fuel.

12. A process (1, 2) according to any preceding Claim, further comprising Step vi. purifying the liquid phase by contacting the liquid phase with one or more of activated carbon or clay, e.g. bentonites and/or acid activated clay, wherein Step vi. may be performed after Step iii. and before Step v and/or wherein the activated carbon and/or clay may be removed via filtration.

13. A process (1, 2) according to any preceding Claim, wherein Step v. of the process (1, 2) comprises acidifying the liquid phase to produce terephthalic acid using an acid, for example hydrochloric acid, e.g. concentration HCl.

14. A process (1, 2) according to any preceding Claim, wherein the pure terephthalic acid is collected via filtration.

15. A process (1, 2) according to any preceding Claim, further comprising Step vii. torrefaction of the mixture comprising char, wherein Step vii. may be performed after Step ii. and before Step iii. of the process (1, 2) and/or wherein Step vii. may be performed at a temperature between 200 to 280 °C, e.g. between 220 to 260°C, or between 240 to 250 °C and/or wherein Step vii. may be performed for less than one hour, e.g. 30 minutes.

## Patentansprüche

1. Vorgang (1, 2) zum Verarbeiten von Ausgangsmaterial, das Baumwollfasern und PET-Fasern enthält, wobei der Vorgang (1, 2) Folgendes umfasst:
i. Erhitzen einer Suspension von Ausgangsmaterial, das Baumwollfasern und PET-Fasern enthält, in Wasser bei einer Temperatur über 180 °C, um eine Mischung zu produzieren, die Kohle umfasst,
ii. Entfernen des Wassers aus der Mischung, die Kohle umfasst,
iii. Zugeben einer Base zu der Mischung, die Kohle umfasst, um ein Terephthalatsalz in einer flüssigen Phase zu produzieren,
iv. Entfernen der Kohle aus der flüssigen Phase, um getrocknete Kohle zu produzieren,
v. Ansäuern der flüssigen Phase, um Terephthalsäure zu produzieren.

2. Vorgang (1, 2) nach Anspruch 1, ferner umfassend Zerkleinern des Ausgangsmaterials vor Schritt i., z. B. in Stücke mit einer Länge und/oder einem Durchmesser von weniger als 50 mm.

3. Vorgang (1, 2) nach einem vorhergehenden Anspruch, wobei das Verhältnis von Wasser zu Ausgangsmaterial zwischen 4:1 und 8:1 (Gew./Gew.), z. B. 4:1, 5:1, 6:1, 7:1 oder 8:1 (Gew./Gew.), Wasser zu Ausgangsmaterial liegt.

4. Vorgang (1, 2) nach einem vorhergehenden Anspruch, wobei Schritt i. Erhitzen einer Suspension von Ausgangsmaterial, das Baumwollfasern und PET-Fasern enthält, in Wasser bei einer Temperatur von 180 bis 240 °C umfasst.

5. Vorgang (1, 2) nach einem vorhergehenden Anspruch, wobei Schritt i. ferner Erhitzen einer Suspension von Ausgangsmaterial, das Baumwollfasern und PET-Fasern enthält, in Wasser unter einem Druck von mehr als 2,0 x 10⁶ Pa (20 bar), z. B. 2,3 x 10⁶ Pa (23 bar), umfasst.

6. Vorgang (1, 2) nach einem vorhergehenden Anspruch, wobei Schritt ii. Entfernen des Wassers aus der Mischung, die Kohle umfasst, über Filtration umfasst.

7. Vorgang (1, 2) nach einem vorhergehenden Anspruch, ferner umfassend Zurückgewinnen von Ethylenglycol aus dem in Schritt ii. des Vorgangs (1, 2) entfernten Wasser, z. B. über Destillation.

8. Vorgang (1, 2) nach einem vorhergehenden Anspruch, wobei Schritt iii. Zugeben einer Base, die Ammoniak und/oder Ammoniumhydroxid umfasst, zu der Mischung, die Kohle umfasst, umfasst, um Diammoniumterephthalat in einer flüssigen Phase zu produzieren, wobei Schritt iii. bei Raumtemperatur durchgeführt werden kann, z. B. zwischen 0 und 40 °C oder zwischen 5 und 35 °C oder zwischen 10 und 30 °C oder zwischen 15 und 25 °C.

9. Vorgang (1, 2) nach einem vorhergehenden Anspruch, wobei Schritt iv. Filtern der Kohle aus der flüssigen Phase umfasst, zum Beispiel unter Verwendung einer Filterpresse.

10. Vorgang (1, 2) nach einem vorhergehenden Anspruch, ferner umfassend Trocknen der Kohle, um einen festen Brennstoff zu produzieren.

11. Vorgang (1, 2) nach einem vorhergehenden Anspruch, ferner umfassend Bilden von Pellets und/oder Briketts aus der Kohle, um einen festen Brennstoff zu bilden.

12. Vorgang (1, 2) nach einem vorhergehenden Anspruch, ferner umfassend Schritt vi. Reinigen der flüssigen Phase durch Inkontaktbringen der flüssigen Phase mit einem oder mehreren von Aktivkohle oder Ton, z. B. Bentoniten und/oder säureaktiviertem Ton, wobei Schritt vi. nach Schritt iii. und vor Schritt v durchgeführt werden kann und/oder wobei die Aktivkohle und/oder der Ton über Filtration entfernt werden können.

13. Vorgang (1, 2) nach einem vorhergehenden Anspruch, wobei Schritt v. des Vorgangs (1, 2) Ansäuern der flüssigen Phase, um Terephthalsäure zu produzieren, unter Verwendung einer Säure umfasst, zum Beispiel Salzsäure, z. B. konzentrierter HCl.

14. Vorgang (1, 2) nach einem vorhergehenden Anspruch, wobei die reine Terephthalsäure über Filtration gesammelt wird.

15. Vorgang (1, 2) nach einem vorhergehenden Anspruch, ferner umfassend Schritt vii. Röstung der Mischung, die Kohle umfasst, wobei Schritt vii. nach Schritt ii. und vor Schritt iii. des Vorgangs (1, 2) durchgeführt werden kann und/oder wobei Schritt vii. bei einer Temperatur zwischen 200 und 280 °C durchgeführt werden kann, z. B. zwischen 220 und 260 °C oder zwischen 240 und 250 °C, und/oder wobei Schritt vii. weniger als eine Stunde lang durchgeführt werden kann, z. B. 30 Minuten.

## Revendications

1. Procédé (1, 2) permettant de traiter une charge d'alimentation contenant des fibres de coton et des fibres de PET, le procédé (1, 2) comprenant :
i. le chauffage d'une suspension d'une charge d'alimentation contenant des fibres de coton et des fibres de PET dans l'eau à une température supérieure à 180°C pour produire un mélange comprenant un produit de carbonisation ;
ii. le retrait de l'eau du mélange comprenant le produit de carbonisation ;
iii. l'ajout d'une base au mélange comprenant le produit de carbonisation pour produire un sel de téréphtalate dans une phase liquide ;
iv. le retrait du produit de carbonisation de la phase liquide pour produire un produit de carbonisation sec ; et
v. l'acidification de la phase liquide pour produire de l'acide téréphtalique.

2. Procédé (1, 2) selon la revendication 1, comprenant en outre le déchiquetage de la charge d'alimentation avant l'étape i, par ex. en morceaux comportant une longueur et/ou un diamètre inférieur à 50 mm.

3. Procédé (1, 2) selon l'une quelconque des revendications précédentes, le rapport de l'eau à la charge d'alimentation étant compris entre 4:1 à 8:1 (p/p), par ex. 4:1, 5:1, 6:1, 7:1 ou 8:1 (p/p), d'eau à la charge d'alimentation.

4. Procédé (1, 2) selon l'une quelconque des revendications précédentes, ladite étape i. comprenant le chauffage d'une suspension de la charge d'alimentation contenant des fibres de coton et des fibres de PET dans de l'eau à une température comprise entre 180 à 240°C.

5. Procédé (1, 2) selon l'une quelconque des revendications précédentes, ladite étape i. comprenant en outre le chauffage d'une suspension de la charge d'alimentation contenant des fibres de coton et des fibres de PET dans de l'eau sous une pression supérieure à 2,0 x 10⁶ Pa (20 bar), par ex. 2,3 x 10⁶ Pa (23 bar).

6. Procédé (1, 2) selon l'une quelconque des revendications précédentes, ladite étape ii. comprenant le retrait de l'eau du mélange comprenant le produit de carbonisation par filtration.

7. Procédé (1, 2) selon l'une quelconque des revendications précédentes, comprenant en outre la récupération de l'éthylène glycol à partir de l'eau retirée dans l'étape ii. du procédé (1, 2), par ex. par distillation.

8. Procédé (1, 2) selon l'une quelconque des revendications précédentes, ladite étape iii. comprenant l'ajout d'une base comprenant de l'ammoniaque et/ou de l'hydroxyde d'ammonium au mélange comprenant le produit de carbonisation pour produire du téréphtalate de diammonium dans une phase liquide, ladite étape iii. pouvant être effectuée à température ambiante, par ex. entre 0 à 40°C, ou entre 5 à 35°C, ou entre 10 à 30°C, ou entre 15 à 25°C.

9. Procédé (1, 2) selon l'une quelconque des revendications précédentes, ladite étape iv. comprenant la filtration du produit de carbonisation à partir de la phase liquide, par exemple à l'aide d'un filtre-presse.

10. Procédé (1, 2) selon l'une quelconque des revendications précédentes, comprenant en outre le séchage du produit de carbonisation pour produire un combustible solide.

11. Procédé (1, 2) selon l'une quelconque des revendications précédentes, comprenant en outre la formation de pastilles et/ou de briquettes à partir du produit de carbonisation pour former un combustible solide.

12. Procédé (1, 2) selon l'une quelconque des revendications précédentes, comprenant en outre l'étape vi. de purification de la phase liquide par la mise en contact de la phase liquide avec un ou plusieurs éléments parmi le charbon actif ou l'argile, par ex. les bentonites et/ou une argile activée par acide, ladite étape vi. pouvant être effectuée après l'étape iii. et avant l'étape v et/ou ledit charbon actif et/ou ladite argile étant retiré par filtration.

13. Procédé (1, 2) selon l'une quelconque des revendications précédentes, ladite étape v. du procédé (1, 2) comprenant l'acidification de la phase liquide pour produire de l'acide téréphtalique à l'aide d'un acide, par exemple l'acide chlorhydrique, par ex. de l'HCl à une concentration.

14. Procédé (1, 2) selon l'une quelconque des revendications précédentes, ledit acide téréphtalique pure étant recueilli par filtration.

15. Procédé (1, 2) selon l'une quelconque des revendications précédentes, comprenant en outre l'étape vii. de torréfaction du mélange comprenant le produit de carbonisation, ladite étape vii. étant effectuée après l'étape ii. et avant l'étape iii. du procédé (1, 2) et/ou ladite étape vii. pouvant être effectuée à une température comprise entre 200 à 280°C, par ex. entre 220 à 260°C ou entre 240 à 250°C et/ou ladite étape vii. pouvant être effectuée pendant moins d'une heure, par ex. 30 minutes.
